# EUROPÄISCHE PATENTANMELDUNG

(11) **EP 0 745 608 A2**
(43) Veröffentlichungstag der Anmeldung: **04.12.1996**
(21) Anmeldenummer: 96108140.3
(22) Anmeldetag: 22.05.1996
(51) Int. Cl.: C07H 15/04, C07H 3/04, C07H 3/06

(54) **Neuartige Glukose- und Sophoroselipide, ein Verfahren zu deren Herstellung un deren Verwendung**

(30) Priorität: 29.05.1995 DE 19518982
(71) Anmelder: HOECHST AKTIENGESELLSCHAFT, 65929 Frankfurt am Main (DE)
(72) Erfinder: Wullbrandt, Dieter, Dr., 65719 Hofheim (DE); Giani, Carlo, Dr., 60956 Frankfurt (DE); Brakemeier, Andreas, DCh., 38114 Braunschweig (DE); Lang, Siegmund, Dr., 38126 Braunschweig (DE); Wagner, Fritz, Prof., 38124 Braunschweig (DE)

(57) **Zusammenfassung**

Die vorliegende Erfindung betrifft Glucose- und Sophoroselipide der Formel I, 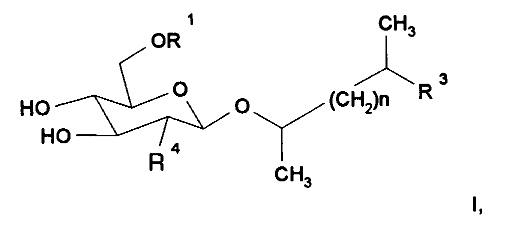 worin
- R⁴: -OH oder eine Gruppe der Formel II, 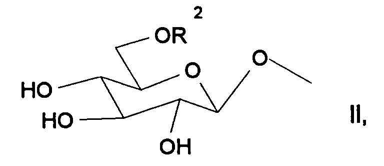
- n: eine ganze Zahl von 2 bis 26,
- R¹ und R²: unabhängig voneinander H oder 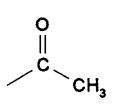 und
- R³: für den Fall, daß R⁴ -OH bedeutet,
H oder -OH und
für den Fall, daß R4 eine Gruppe der Formel II bedeutet,
H, -OH oder eine Gruppe der Formel III, 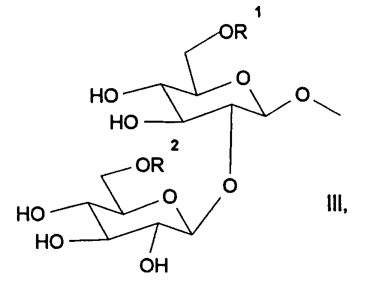
bedeuten, ein fermentatives Verfahren zu deren Gewinnung sowie deren Verwendung als Tenside.

## Beschreibung

Die vorliegende Erfindung betrifft neuartige Glucose- und Sophoroselipide, ein Verfahren zu deren fermentativen Herstellung sowie deren Verwendung als Tenside.

Mikrobielle Glycolipide sind seit einigen Jahren als Biotenside mit vielfältigen Anwendungsperspektiven in der Kosmetik, dem Wasch- und Reinigungsmittelsektor, dem Lebensmittelbereich und dem Umweltschutz bekannt. Sie werden von Bakterien, Hefen oder Pilzen bei Wachstum auf langkettigen Erdölprodukten, auf pflanzlichen Ölen und Fetten oder deren Derivaten oder auf Mono- und Oligosacchariden gebildet. Eine gezielte Modifikation der molekularen Strukturen dieser Produkte durch Variation der Kohlenstoffquelle konnte bisher nur in geringem Maße erreicht werden. Seit seiner Entdeckung im Jahre 1961 gehört das Sophoroselipid zu den intensiv erforschten mikrobiellen Glycolipiden [P. A. Gorin, J. F. T. Spencer und A. P. Tulloch; Cand. J. Chem, 39 (1961), 846 - 855].

Das Sophoroselipid läßt sich verschiedenen Berichten zufolge durch verschiedene Hefen der Gattung Candida (Torulopsis) als Sekundärmetabolit mittels eines Substrats aus den oben angegebenen Kohlenstoffquellen herstellen. Als geeignete Hefestämme sind Candida bombicola, Candida bogoriensis, Candida magnoliae, Candida gropengiesseri und Candida apicola beschrieben [R. Hommel, Biodegradation, 1, (1991), 107].

Die von der Gattung Candida gebildeten Sophoroselipide verfügen über eine nachfolgend abgebildete Struktur (1).

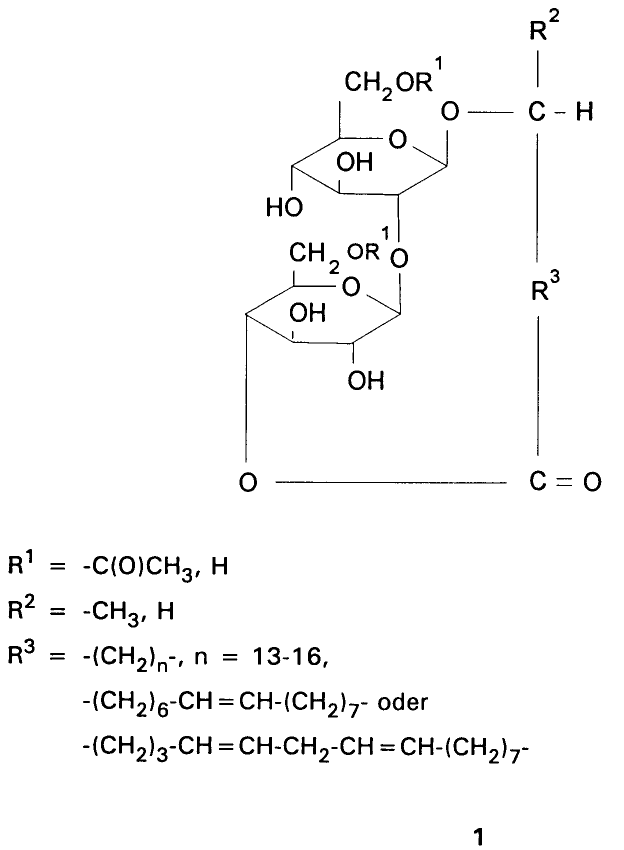

Neben diesem lactonischen Hauptprodukt mit sowohl glycosidischer als auch esterartig gebundener Hydroxyfettsäure werden in geringen Anteilen auch nicht cyclisierte Zwischenstufen gefunden. Die Hydroxyfettsäure kann je nach eingesetztem Substrat gesättigt, einfach aber auch mehrfach ungesättigt sein. Darüber hinaus sind die 6'-O- und 6''-O-Positionen der Glucose-Einheiten in unterschiedlichem Maß acetyliert. Bei diesen Sophoroselipiden beobachtet man nur geringe Unterschiede in den Fettsäuren der Nebenkette.

Zur Erzeugung eines Glycolipids mit amphiphiler Struktur, d. h. hoher Grenzflächenaktivität, müssen die Sophoroselipid-Lactone in die Sophoroselipid-Ester bzw. Amide über aufwendige Synthese- und Aufreinigungstufen umgewandelt werden [S. Inoue, et al. US Patent. 4,215,213, 1990; Y. Ishigami, JP-Anmeldung: Toku Kai Hei 6-100581].

Überraschenderweise wurde nun gefunden, daß bei der Fermentation von Hefen der Gattung Candida unter Einsatz von 2-Alkanolen anstelle der pflanzlichen Öle und Fette, Fettsäuren bzw. deren Alkylester neue, nicht cyclisierte Glucose- und Sophoroselipide mit grenzflächenaktiven Eigenschaften zugänglich sind.

Die Erfindung betrifft somit eine Verbindung der Formel I, 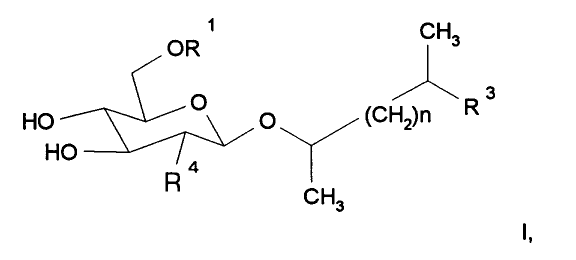 worin bedeuten
- R⁴: -OH oder eine Gruppe der Formel II, 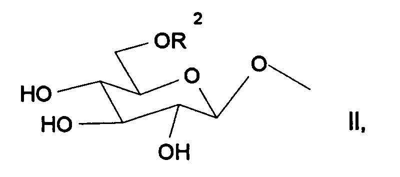
- n: eine ganze Zahl von 2 bis 26,
- R¹ und R²: unabhängig voneinander H oder 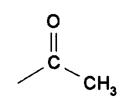 und
- R³: für den Fall, daß R⁴ -OH bedeutet,
H oder -OH und
für den Fall, daß R⁴ eine Gruppe der Formel II bedeutet,
H, -OH oder eine Gruppe der Formel III,

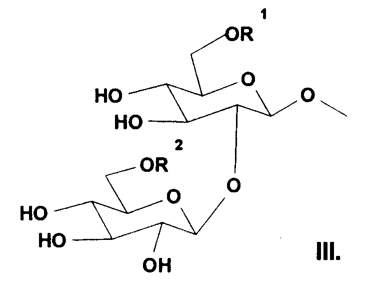

Bevorzugte Ausgestaltungen der vorliegenden Erfindung sind nachfolgend dargestellt.
1. Eine Verbindung der Formel 1, worin R⁴ eine Gruppe der Formel II bedeutet, wobei
   R¹ und R² gemeinsam -C(O)CH₃,
   R¹ H und R² -C(O)CH₃,
   R¹ -C(O)CH₃ und R² H oder
   R¹ und R² H bedeuten und
   R³ H, -OH oder eine Gruppe der Formel III darstellt und wobei n eine ganze Zahl von 6 bis 14, vorzugsweise 8 oder 10 bedeutet.
2. Eine Verbindung der Formel 1, worin R⁴ -OH bedeutet, wobei R¹ -C(O)CH₃ oder H, R³ -OH oder vorzugsweise H bedeutet und wobei n eine ganze Zahl von 6 bis 14, vorzugsweise 8 oder 10 bedeutet.

Die vorliegende Erfindung betrifft ferner ein Verfahren zur Herstellung einer Verbindung der Formel I, wobei die Variablen R¹, R², R³, R⁴ und n die genannten Bedeutungen haben, welches sich dadurch auszeichnet, daß eine Hefe mit der Fähigkeit, Sophoroselipide in Form eines Lactons in den Kulturüberstand zu sezernieren, in einem Kulturmedium fermentiert wird, welches neben einer weiteren Kohlenstoffquelle, wie Glycerin, Succinat oder Mono-, Di- oder Trisaccharide, ein 2-Alkanol mit einer Kettenlänge von 6 bis 30 Kohlenstoffatomen enthält, wonach die Verbindung aus der Kulturlösung isoliert und gegebenfalls einer alkalischen Hydrolyse unterworfen wird.

Vorzugsweise beträgt die Kettenlänge des 2-Alkanols 10 bis 18 Kohlenstoffatome, womit sich eine Verbindung der Formel I herstellen läßt, in welcher n eine ganze Zahl von 6 bis 14 bedeutet, besonders bevorzugt wird als Alkanol 2-Dodecanol oder 2-Tetradecanol eingesetzt, wodurch sich eine Verbindung der Formel I gemäß den oben genannten Nummern 1 oder 2 (mit n = 8 oder n = 10) herstellen läßt.

Bei dem Verfahren gemäß der vorliegenden Erfindung können alle Hefestämme fermentiert werden, welche die in der Literatur beschriebenen Sophoroselipide in Lactonform (1) in den Kulturüberstand sezernieren.

Für das erfindungsgemäße Verfahren ist eine Hefe der Gattung Candida besonders geeignet, vorzugsweise wird Candida bombicola, Candida bogoriensis, Candida magnoliae, Candida gropengiesseri oder Candida apicola fermentiert.

Als Kohlenstoffquelle sind Glucose oder Saccharose besonders geeignet.

Die vorliegende Erfindung betrifft ferner die Verwendung einer Verbindung der Formel I, in der R³ H oder OH bedeutet, als Tensid.

Im folgenden wird die Erfindung detailliert beschrieben. Ferner wird sie durch den Inhalt der Patentansprüche bestimmt.

Durch den Einsatz von 2-Alkanolen mit Kettenlängen von C6 bis C30 als hydrophobe Kohlenstoffquelle neben einer weiteren Kohlenstoffquelle wie z. B. Glycerin, Succinat oder Mono-, Di- und Trisaccharide wie z. B. Saccharose, Mannose, Maltose, Fructose, Glucose sowie D-Mannitol oder andere Zuckeralkohole, vorzugsweise Glucose oder Saccharose, gelingt die Gewinnung von Glucose- und Sophoroselipiden mit einer Variation der Struktur des hydrophoben Molekülteils. Die aufgereinigten Produkte enthalten jeweils nur Lipidkomponenten der Kettenlänge des jeweils eingesetzten Substrates, wobei letzteres entweder direkt in das Glycolipid (Verbindungen der Strukturen 2 bzw. 5 mit R³ = H) eingebaut wird oder nach einer (ω -1)-Hydroxylierung als Alkandiol über eine glycosidische Bindung an die Zuckerkomponente gebunden ist (Verbindungen der Struktur 3 bzw. 5 mit R³ = OH).

Auf diese Weise entstehen ausschließlich nichtionische Tenside mit typischer Tensidstruktur.
Die Verbindungen der Struktur 3 können durch eine anschließende mikrobielle Umsetzung mit einer zweiten Zucker-/Sophorose-Einheit in die Verbindungen mit der Struktur 4 überführt werden. Diese sehr polaren Verbindungen verfügen über nur geringfügige tensidische Eigenschaften.

Die Produkte sind im Zuckerteil in der 6'-O- und 6''-O-Position der Glucoseeinheit nicht, mono- oder diacetyliert. Die Gewinnung der Verbindungen entsprechend den Strukturen 2, 3, 4 und 5, jeweils mit n = 2 bis 26 sind durch Variation der Kettenlänge der eingesetzten 2-Alkanole (C6 bis C30) möglich.

Demgemäß läßt sich beispielsweise beim Einsatz von
- 2-Hexanol eine Verbindung der Formel I mit n = 2,
- 2-Octanol eine Verbindung der Formel I mit n = 4,
- 2-Decanol eine Verbindung der Formel I mit n = 6,
- 2-Undecanol eine Verbindung der Formel I mit n = 7,
- 2-Pentadecanol eine Verbindung der Formel I mit n = 11,
- 2-Octadecanol eine Verbindung der Formel I mit n = 14,
- 2-Eicosanol eine Verbindung der Formel I mit n = 16
oder beim Einsatz von 2-Triacontanol eine Verbindung der Formel I mit n = 26 herstellen.

Die 2-Alkanole der Kettenlänge C6 bis C30 sind teils käuflich zu erwerben, teils lassen sie sich aus den entsprechenden, wohlfeilen 2-Alkanonen, 1-Alkenen oder den Aldehyden der Kettenlänge Cₙ₋₁ (Cₙ = 6 bis 30) darstellen (Organikum, 16. Auflage, VEB-Deutscher Verlag der Wissenschaften 1986 oder anderes entsprechendes Lehrbuch der angewandten organischen Chemie).

So können die entsprechenden 2-Alkanone durch katalytische Hydrierung unter Raney-Nickel Katalyse in methanolischer Lösung (Organikum 1986, S. 432) oder durch Reduktion mit Lithiumaluminiumhydrid in etherischer Lösung (Organikum 1986, S. 494) in die C₆-C₃₀-2-Alkanole überführt werden.

Die mit einem Quecksilbersalz katalysierte Anlagerung von Wasser an die Doppelbindung der 1-Alkene der Kettenlängen C6 bis C30, die sogenannte Oxymercurierungsreaktion, führt ebenfalls zu den entsprechenden sekundären Alkanolen, den C6-C30-2-Alkanolen (Organikum 1986, S. 257).

Ferner liefert die Reduktion von Aldehyden der Kettenlänge Cₙ₋₁ (n = 6 bis 30) mittels Methyllithium oder mittels eines Methylmagnesiumbromid-Grignard-Reagenzes in etherischer Lösung das gewünschte 2-Alkanol der Kettenlänge Cₙ (n = 6 bis 30) (Organikum 1986, S. 499). 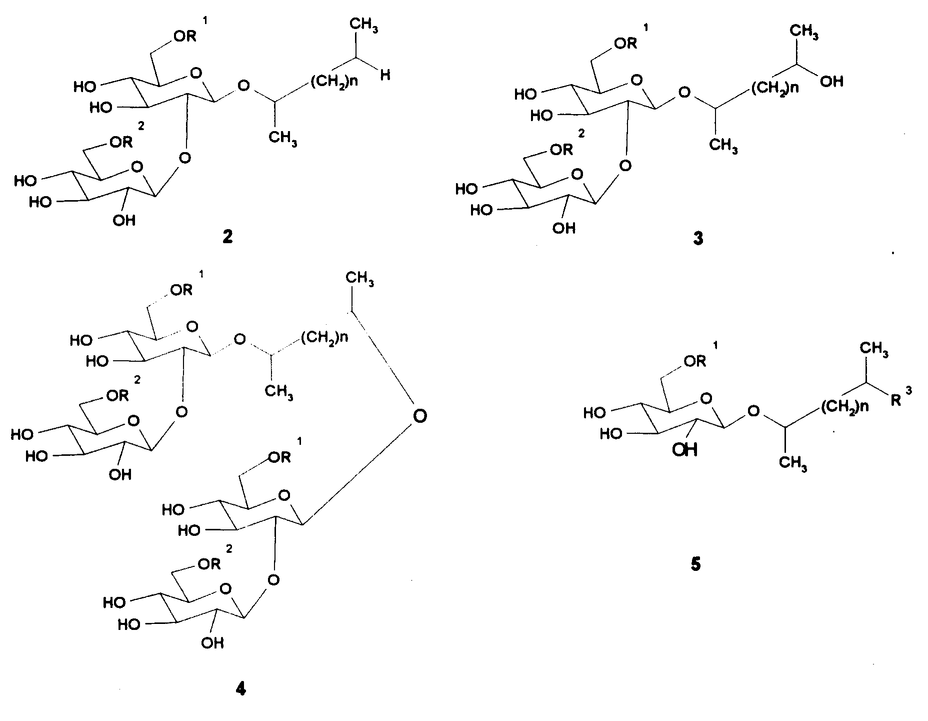
- 2, 3, 4:: n = 2 - 26; R¹, R² = H oder -C(O)CH₃
- 5:: n = 2 - 26; R¹ - H oder -C(O)CH₃; R³ = H oder -OH
- 2d:: R¹ = R² = H (s.Beispiele 1 und 2: n = 10; Beispiel 3: n = 8)
- 2e:: R¹ = -C(O)CH₃, R² = H (s. Beispiel 1: n = 10; Beispiel 3: n = 8)
- 2f:: R¹ = R² = C(O)CH₃ (s.Beispiel 1: n = 10; Beispiel 3: n = 8)
- 3g:: R¹ = R² = H (s. Beispiel 2: n = 10; Beispiel 3: n = 8)
- 3b:: R¹ = -C(O)CH₃, R² = H (s. Beispiel 1: n = 10; Beispiel 3: n = 8)
- 3c:: R¹ = R² = -C(O)CH₃ (s. Beispiel 1: n = 10; Beispiel 3: n = 8)
- 4h:: R¹ = R² = H (s. Beispiel 2: n = 10; Beispiel 3: n = 8)
- 4a:: R¹ = R² = -C(O)CH₃ (s. Beispiel 1: n = 10; Beispiel 3: n = 8)
- 5a:: R¹ = H; R³ = H (s. Beispiel 3: n = 8)

Am Ende der Fermentation wird die Kulturlösung mit einer Lauge neutralisiert und die gebildeten Sophoroselipide der Strukturen 2 - 5 durch erschöpfende Extraktion mit einem geeigneten Lösungsmittel wie z. B. Carbonsäureester, wie Essigsäureethylester, Essigsäurebutylester oder Ether, wie tert-Butylmethylether und Diethylether oder sonstige, dem Fachmann bekannte Lösungsmittel gewonnen.

Die so gewonnenen Sophoroselipide bewirken bereits bei deutlich erhöhter Löslichkeit relativ zu den klassischen Produkten eine größere Erniedrigung der Oberflächenspannung von Wasser.

Durch alkalische Verseifung mit Laugen (z. B. wäßrige NaOH) bzw. Alkanolaten (z. B. Natriummethanolat) können die in 6'-O- und 6''-O-Position acetylierten Produkte in die entsprechenden Hydroxyverbindungen umgewandelt werden.

Als Mikroorganismen können alle Hefestämme fermentiert werden, die die in der Literatur beschriebenen Sophoroselipide in Form des Lactons in den Kulturüberstand sezernieren. Vorzugsweise eingesetzte Hefestämme sind Candida bombicola, Candida bogoriensis, Candida magnoliae, Candida gropengiesseri und Candida apicola.

Die eingesetzten Produzentenstämme werden in einem Medium mit 2-Alkanolen mit einer Kettenlänge von C6 bis C30, vorzugsweise C10 bis C18, fermentiert. Die Konzentration an 2-Alkanolen kann zu Beginn der Fermentation eingestellt werden bzw. durch kontinuierliche Nachdosierung entsprechend der Umsatzrate gewählt werden. Zur Bereitstellung einer zusätzlichen Kohlenstoffquelle hat sich die Verwendung von Zuckern, vorzugsweise Glucose oder Saccharose, bewährt. Das Medium sollte außer der Kohlenstoffquelle noch eine oder mehrere Stickstoffquellen, Sulfat und Magnesium sowie Kalium-, Natrium-, Calcium- und Chloridionen, eine oder mehrere Phosphatquellen sowie ein das Wachstum förderndes komplexes Substrat wie z. B. Hefeextrat enthalten. Der Zucker wird in Konzentrationen von 30 bis 200 g/l Nährlösung verwendet, wobei Konzentrationen zwischen 80 und 120 g/l zu bevorzugen sind. Als Stickstoffquelle können dem Fachmann bekannte Stickstoffquellen, wie z. B. Harnstoff, Ammoniumchlorid oder Ammoniumsulfat in Konzentrationen von 0,1 bis 5 g/l Nährlösung verwendet werden, wobei vorzugsweise eine Konzentration von 0,5 - 2,5 g/l gewählt wird.

Als Phosphorquelle und zur Pufferung des Mediums wird ein 0,001 bis 0,1 molarer Natriumphosphat- oder Kaliumphosphatpuffer oder eine Mischung der beiden Metallionen eingesetzt.
Der pH Wert der Kulturlösung sinkt während der Fermentationszeit ab.

Zur Isolierung der Produkte wird die Kulturlösung nach Abtrennen der Biomasse durch Zentrifugation oder Filtration mit einer Lauge neutralisiert und mit einem organischen Lösungsmittel bei Bedarf mehrfach extrahiert. Die organischen Phasen werden abgetrennt, vereinigt und über einem Trockenmittel wie z. B. Natriumsulfat getrocknet. Nach Entfernen des Extraktionsmittels im Vakuum wird nach azeotroper Entfernung des Wassers ein gelb-braunes Rohprodukt erhalten.
Die Erfindung wird nachstehend an Ausführungsbeispielen näher erläutert.

### Beispiel 1

Zur Produktion der Biotenside in einem 2 l-Erlenmeyerkolben mit Schikanen werden 400 ml eines Kulturmediums der folgenden Zusammensetzung vorgelegt:

| | |
|---|---|
| Glucose·H₂O | 100 g/l |
| Natriumcitrat·3 H₂O | 5 g/l |
| Hefe-Extrakt | 1 g/l |
| Ammoniumchlorid | 1,54 g/l |
| Kaliumdihydrogenphosphat | 1 g/l |
| Magnesiumsulfat·7 H₂O | 0,7 g/l |
| Natriumchlorid | 0,5 g/l |
| Calciumchlorid·2 H₂O | 0,27 g/l |
| Dikaliumhydrogenphosphat·3 H₂O | 0,16 g/l |

Das Medium wird mit dem Stamm *Candida bombicola* ATCC 22214 beimpft und auf einer Rotations-Schüttelmaschine bei 100 Upm und einer Temperatur von 30 °C inkubiert. Nach einer Kultivierungsdauer von 24, 48 und 72 h werden der Kulturlösung unter aseptischen Bedingungen jeweils 10 g/l 2-Tetradecanol zugesetzt. Die Kulturführung zwischen und nach den Zugaben des Alkohols erfolgt unter unveränderten Bedingungen. Über den gesamten Bereich der Kultivierung sinkt der pH-Wert der Kultursuspension ab. Nach einem Kultivierungszeitraum von 7 d ist die angebotene Menge des Alkohols umgesetzt, die Kultivierung wird daraufhin abgebrochen.

Zur Isolierung der Produkte wird die Kultursuspension mit 1 N Natronlauge neutralisiert und anschließend mit Essigsäureethylester erschöpfend extrahiert. Die organischen Phasen werden abgetrennt, vereinigt und über wasserfreiem Natriumsulfat getrocknet. Nach dem Abtrennen des Trockenmittels über einen Papierfilter wird das Lösungsmittel bei vermindertem Druck am Rotationsverdampfer abdestilliert. Das zurückbleibende, hochviskose Rohprodukt wird zur azeotropen Entfernung des gebundenen Wassers mit n-Butanol versetzt. Anschließend wird bei vermindertem Druck erneut vollständig abdestilliert. Das erstarrte, nahezu wasserfreie, gelb-braune Rohprodukt wird mit dem doppelten Volumen an trockenem Diethylether überschichtet und einige Stunden bei Raumtemperatur stehengelassen. Dabei entfärbt sich das Rohprodukt unter leichter Volumensvergrößerung zu einem fast farblosen Feststoff, dem eine orange-braune Etherphase übersteht. Nach kräftigem Aufschütteln wird der Feststoff über einen Papierfilter abgetrennt, mit wenig Ether gewaschen und getrocknet. Das noch etwas klebrige Glycolipidgemisch kann durch Waschen mit Eiswasser weiter standardisiert werden. Es wird in einer Ausbeute von 23 g/l erhalten.
Das so aufbereitete Glycolipidgemisch läßt sich an silyliertem Kieselgel (RP-8) im Laufmittelgemisch Methanol/Wasser 80 : 20 (v/v) dünnschichtchromatographisch in 6 Einzelsubstanzen (Verbindungen 4a, 3b, 3c, 2d, 2e und 2f, jeweils mit n = 10) mit charakteristischen R_{f}-Werten trennen. Mittels Kernresonanzspektroskopie, FAB- bzw. TOF-SI-Massenspektrometrie sowie einer kombinierten gaschromatographischmassenspektrometrischen Analyse des hydrophoben Molekülteils (nach saurer Methanolyse) der Verbindungen kann belegt werden, daß den Einzelverbindungen die dargestellten molekularen Strukturen 2, 3 und 4 (jeweils mit n = 10) zugrundeliegen. Hauptprodukt der Kultivierung ist die Verbindung mit der Struktur 2f (n = 10).

### Spektroskopische Daten:

4a:
   - ¹³C-NMR (CD₃OD, 100 MHz, ppm):: 174.20 (s, -OCO-CH₃)
   107.16/104.15 (2d, C-1'/ C-1'')
   66.49/66.30 (2t, C-6'/C-6'')
   23.39 (q, C-1/14)
   22.48/22.29 (q, -OCO-CH₃)
   - FAB-MS (Matrix NBA, pos., m/z):: 1069 (100, [M+Na]⁺)
   - Lipidkomponente: GC-MS (El, m/z):: 212 (2, [M-H₂O]⁺)
   - 2, 13-Tetradecandiol: 45 (100, [CH₃CHOH]⁺)
3b:
   - TOF-SIMS (pos., m/z):: 619 (100, [M+Na]⁺)
   703 (34, [M+¹⁰⁷Ag]⁺)
   705 (27, [M+¹⁰⁹Ag]⁺)
   - Lipidkomponente: GC-MS (El, m/z):: 212 (2, [M-H₂O]⁺)
   - 2, 13-Tetradecandiol: 45 (100, [CH₃CHOH]⁺)
3c:
   - ¹³C-NMR (CD₃OD, 100 MHz, ppm):: 174.22 (s, -OCO-CH₃)
   107.19/104.17 (2d, C-1'/C-1'')
   66.49/66.30 (2t, C-6'/C-6'')
   25.05 (q, C-14), 23.38 (q, C-1)
   22.44/22.279 (q, -OCO-CH₃)
   - FAB-MS (Matrix NBA, pos., m/z):: 661 (100, [M+Na]⁺)
   - Lipidkomponente: GC-MS (El, m/z):: 212 (2, M-H₂O]⁺)
   - 2, 13-Tetradecandiol: 45(100, [CH₃CHOH]⁺)
2d:
   - ¹³C-NMR (CD₃OD, 100 MHz, ppm):: 104.69/102.80 (2d, C-1'/ C-1'')
   63.12/62.80 (2t, C-6'/C-6'')
   21.94 (q, C-1)
   - TOF-SIMS (pos., m/z):: 561 (100, [M+Na]⁺)
   645 (18, [M+¹⁰⁷Ag]⁺)
   647 (17, [M+¹⁰⁹Ag]⁺)
   - Lipidkomponente: GC-MS (El, m/z):: 196 (1, [M-H₂O]⁺)
   - 2-Tetradecanol: 45 (100, [CH₃CHOH]⁺)
2e:
   - TOF-SIMS (pos., m/z):: 603 (100, [M+Na]⁺)
   687 (28, [M+¹⁰⁷Ag]⁺)
   689 (28, [M+¹⁰⁹Ag]⁺)
   - Lipidkomponente: GC-MS (El, m/z):: 196 (1, [M+H₂O]⁺)
   - 2-Tetradecanol: 45 (100, [CH₃CHOH]⁺)
2f:
   - TOF-SIMS (pos., m/z):: 645 (100, M+Na]⁺)
   729 (32, [M+¹⁰⁷Ag]⁺)
   731 (30, [M+¹⁰⁹Ag]⁺)
   - Lipidkomponente: GC-MS (El, m/z):: 196 (1, [M-H₂O]⁺)
   - 2-Tetradecanol: 45 (100, [CH₃CHOH]⁺)

### Beispiel 2

Zur basischen Hydrolyse des nach Beispiel 1 gewonnenen Sophoroselipids werden 10 g des Glycolipidgemisches in 300 ml 1N Natronlauge gelöst und unter Rühren für 12 h refluxiert.
Anschließend wird die Reaktionslösung mit konzentrierter Salzsäure neutralisiert und auf 4 °C abgekühlt. Der sich abscheidende Niederschlag wird über einen Papierfilter abgetrennt, mit 100 ml eiskaltem Wasser gewaschen und zur Umkristallisation (60 °C → 4 °C) erneut in 300 ml Wasser aufgenommen. Das kristallisierte Produkt wird erneut abfiltriert und getrocknet. Mittels chromatographischer Trennung an silyliertem Kieselgel (RP-8) im Laufmittelsystem Methanol/Wasser 80 : 20 (v/v) kann der Feststoff in zwei Substanzen getrennt werden. Die spektroskopischen Untersuchungen der aufgetrennten Substanzen belegen, daß die unpolarere Komponente die Struktur 2d (mit n = 10) aufweist. Die polarere Komponente, ist die Verbindung mit der Struktur 3g (mit n = 10).

Der flüssige Teil der Reaktionsmischung (1. Filtrat) wird zweimal mit dem doppelten Volumen Essigsäureethylester extrahiert. Die vereinigten organischen Phasen werden über wasserfreiem Natriumsulfat getrocknet, anschließend das Extraktionsmittel unter vermindertem Druck abdestilliert. Der gelb-braune, nichtflüchtige Rückstand wird zur azeotropen Entfernung des gebundenen Wassers mit dem gleichen Volumen n-Butanol versetzt. Nach dem Abdestillieren des Alkohols bleibt ein bei Raumtemperatur erstarrendes Rohprodukt zurück. Es enthält ein weiteres, sehr polares, chromatographisch abtrennbares Sophoroselipid. Es hat die Struktur 4h (mit n = 10) und entspricht strukturell einem desacetylierten 4a (n = 10). Insgesamt werden 6 g desacetyliertes Glycolipid erhalten, der Hauptanteil entfällt auf das Produkt der Formel 2d (mit n = 10).

### Spektroskopische Daten:

2d:
   - siehe Beispiel 1:
3g:
   - ¹³C-NMR (CD₃OD, 100 MHz, ppm):: 104.58/102.70 (2d, C-1'/C-1'')
   63.01/62.69 (2t, C-6'/C-6'')
   23.79 (q, C-14), 21.84 (q, C-1)
   - FAB-MS (neg., m/z):: 553 (100, [M-H]⁻)
   - Lipidkomponente: GC-MS (El, m/z):: 212 (2, M-H₂O]⁺)
   - 2, 13-Tetradecandiol: 45(100, [CH₃CHOH]⁺)
4h:
   - ¹³C-NMR (CD₃OD, 100 MHz, ppm):: 104.16/102.15 (2d, C-1'/C-1'')
   63.18/62.12 (2t, C-6'/C-6'')
   22.39 (q, C-1/14)
   - FAB-MS (Matrix NBA, pos., m/z):: 901 (100, [M+Na]⁺)
   - Lipidkomponente: GC-MS (El, m/z):: 212 (2, M-H₂O]⁺)
   - 2, 13-Tetradecandiol: 45(100, [CH₃CHOH]⁺)

### Beispiel 3:

Zur Produktion der Biotenside in einem 500 ml-Erlenmeyerkolben mit Schikanen werden 100 ml eines Kulturmediums der folgenden Zusammensetzung vorgelegt:

| | |
|---|---|
| Glucose·H₂O | 100 g/l |
| Natriumcitrat·3 H₂O | 5 g/l |
| Hefe-Extrakt | 1 g/l |
| Ammoniumchlorid | 1,54 g/l |
| Kaliumdihydrogenphosphat | 1 g/l |
| Magnesiumsulfat·7 H₂O | 0,7 g/l |
| Natriumchlorid | 0,5 g/l |
| Calciumchlorid·2 H₂O | 0,27 g/l |
| Dikaliumhydrogenphosphat·3 H₂O | 0,16 g/l |

Das Medium wird mit der Hefe Candida bombicola ATCC 22214 beimpft und auf einer Rotations-Schüttelmaschine bei 100 Upm und einer Temperatur von 30 °C inkubiert. Nach einer Kultivierungsdauer von 24, 48 und 72 h werden der Kulturlösung unter aseptischen Bedingungen jeweils 10 g/l 2-Dodecanol zugesetzt. Die Kulturführung zwischen und nach den Zugaben des Alkohols erfolgt unter unveränderten Bedingungen. Über den gesamten Bereich der Kultivierung sinkt der pH-Wert der Kultursuspension ab. Nach einem Kultivierungszeitraum von 8 d ist die angebotene Menge des Alkohols umgesetzt, die Kultivierung wird daraufhin abgebrochen.

Zur Isolierung der Produkte wird die Kultursuspension mit 1 N Natronlauge neutralisiert und anschließend mit Essigsäureethylester erschöpfend extrahiert. Die organischen Phasen werden abgetrennt, vereinigt und über wasserfreiem Natriumsulfat getrocknet. Nach dem Abtrennen des Trockenmittels über einen Papierfilter wird das Lösungsmittel bei vermindertem Druck am Rotationsverdampfer abdestilliert. Das zurückbleibende, hochviskose Rohprodukt wird zur azeotropen Entfernung des gebundenen Wassers mit n-Butanol versetzt. Anschließend wird bei vermindertem Druck erneut vollständig abdestilliert. Das erstarrte, nahezu wasserfreie, gelb-braune Rohprodukt wird mit dem doppelten Volumen trockenem Diethylether überschichtet und einige Stunden bei Raumtemperatur stehengelassen. Dabei entfärbt sich das Rohprodukt unter leichter Volumensvergrößerung zu einem fast weißen Feststoff, dem eine orange-braune Etherphase übersteht. Nach kräftigem Aufschütteln wird der Feststoff über einen Papierfilter abgetrennt, mit wenig Ether gewaschen und getrocknet. Das noch etwas klebrige Glycolipidgemiscch wird durch wiederholtes Waschen mit Eiswasser weiter standardisiert. Es wird in einer Ausbeute von 18 g/l erhalten. Es vermag die Oberflächenspannung von Wasser bei 25 °C von 72 auf 29,5 mN/m zu erniedrigen (Knickpunkt der σ/c-Isotherme bei 200 mg/l).

Läßt man eine bei 60 °C gesättigte, wäßrige Lösung dieses Glycolipidgemisches auf Raumtemperatur abkühlen, bilden sich in der Lösung feine, nadelförmige Feststoffpartikel. Die Kristallisation wird bei 4 °C vervollständigt. Die Nadeln werden über einen Papierfilter abgetrennt, das Filtrat wird zur Entfernung des Lösungsmittels und zur Rückgewinnung der nicht kristallisierten Glycolipide gefriergetrocknet.
Die nachfolgende chromatographische Trennung des nichtkristallinen Anteils an silyliertem Kieselgel (RP-8) im Laufmittelgemisch Methanol/Wasser 80 : 20 (v/v) liefert 6 Einzelsubstanzen. Wie die spektroskopische Analyse der Einzelsubstanzen belegt, besitzen diese die Molekülstrukturen 2, 3 und 4 (jeweils mit n = 8) mit einem hydrophoben Molekülteil der Kettenlänge C₁₂. Hauptprodukt der Kultivierung ist auch hier die Verbindung der Struktur 2 f (mit n = 8).

Nach basischer Hydrolyse des Produktgemisches und chromatographischer Trennung analog zu Beispiel 2 lassen sich die Produkte mit den Strukturen 2 d, 3 g und 4 h (jeweils mit n = 8) isolieren.

Wie mittels dünnschichtchromatographischer Untersuchungen an silyliertem Kieselgel (RP-8) im Laufmittelgemisch Methanol/Wasser 80 : 20 (v/v) ermittelt, besteht der kristallisierte Teil des Produktgemisches aus einer weiteren, bisher nicht beschriebenen Reinsubstanz. Kernresonanzspektroskopie, FAB- bzw. TOF-SI-Massenspektrometrie sowie die kombinierte gaschromatographischmassenspektrometrische Analyse des hydrophoben Molekülteils (nach saurer Methanolyse) der Verbindungen belegen das Vorliegen eines Glucoselipids mit der Molekülstruktur 5 a (n = 8).

### Spektroskopische Daten:

5 a:
   - ¹³C-NMR (CD₃OD, 100 MHz, ppm):: 102.36 (d, C-1')
   76.62 (d, C-3')
   76.30 (d, C-5')
   76.10 (d, C-2)
   73.79 (d, C-2')
   70.18 (q, C-4')
   61.30 (t, C-6')
   31.57-24.90 (8t, C-4-11)
   22.22 (t, C-3)
   20.44 (q, C-1)
   12.93 (q, C-12)
   - FAB-MS (Matrix NBA, neg., m/z):: 153 (100, [NBA]⁻)
   347 ( 32, [M-H]⁻)
   501 (26, [M+NBA]⁻)
   695 ( 6, [2M-H]⁻)
   849 ( 1, [2M+NBA]⁻)
   - Lipidkomponente: GC-MS (El, m/z):: 168 (2, [M-H₂O]⁺)
   - 2-Dodecanol: 45 (100, [CH₃CHOH]⁺)

### Beispiel 4

Die oberflächenaktive Wirkung bzw. die tensidischen Eigenschaften der gemäß Beispiel 1 bzw. Beispiel 3 gewonnenen Glycolipidgemische wird durch die Messung ihres Einflusses auf die Oberflächenspannung von Wasser dokumentiert.

Dazu werden wäßrige Lösungen verschiedener Konzentrationen c der Produktgemische hergestellt und deren Oberflächenspannung σ an einem Ringtensiometer (Fa. MGW Lauda, Lauda-Königshofen; Methode ohne Abriß nach de Noüy) gemessen (σ/c-Isothermen).

| Ergebnisse: | | |
|---|---|---|
| c[mg/l] | σ [mN/m] Glycolipidgemisch aus Beispiel 3 | σ [mN/m] Glycolipidgemisch aus Beispiel 1 |
| 500 | 29,5 | 30,8 |
| 300 | 29,6 | 30,8 |
| 200 | 29,8 | 30,9 |
| 100 | 31,1 | 31,6 |
| 50 | 33,0 | 32,8 |
| 10 | 45,2 | 39,3 |
| 5 | 50,8 | 46,2 |
| 1 | 60,4 | 57,0 |
| 0,5 | 65,0 | 60,5 |
| 0,1 | 68,5 | 66,2 |
| 0,01 | 70,3 | 70,0 |
| dest. Wasser | 72,0 | 72,0 |

## Patentansprüche

1. Verbindung der Formel I, 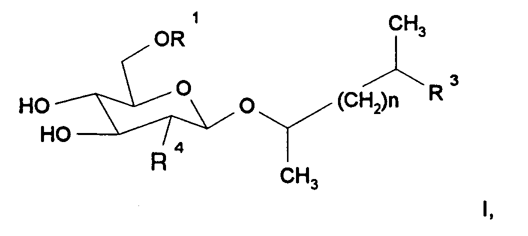 worin bedeuten
R⁴ -OH oder eine Gruppe der Formel II, 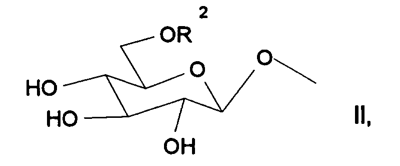
n eine ganze Zahl von 2 bis 26,
R¹ und R² unabhängig voneinander H oder 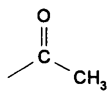 und
R³ für den Fall, daß R⁴ -OH bedeutet, H oder -OH und
für den Fall, daß R⁴ eine Gruppe der Formel II bedeutet,
H, -OH oder eine Gruppe der Formel III,

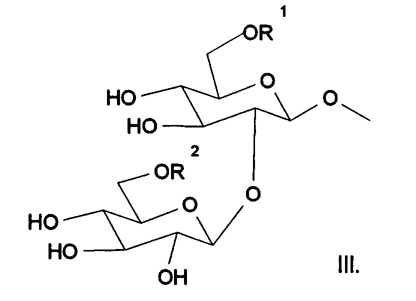

2. Verbindung nach Anspruch 1, dadurch gekennzeichnet, daß R⁴ eine Gruppe der Formel II, 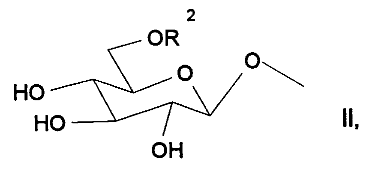 bedeutet.

3. Verbindung nach Anspruch 2, dadurch gekennzeichnet, daß R¹ und R² -C(O)CH₃ bedeuten.

4. Verbindung nach Anspruch 2, dadurch gekennzeichnet, daß R¹ H und R² -C(O)CH₃ bedeutet.

5. Verbindung nach Anspruch 2, dadurch gekennzeichnet, daß R¹ -C(O)CH₃ und R² H bedeutet.

6. Verbindung nach Anspruch 2, dadurch gekennzeichnet, daß R¹ und R² H bedeuten.

7. Verbindung nach einem der Ansprüche 3 bis 6, dadurch gekennzeichnet, daß R³ H bedeutet.

8. Verbindung nach einem der Ansprüche 3 bis 6, dadurch gekennzeichnet, daß R³ -OH bedeutet.

9. Verbindung nach einem der Ansprüche 3 bis 6, dadurch gekennzeichnet, daß R³ eine Gruppe der Formel III, 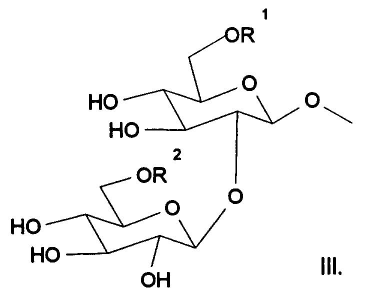 bedeutet.

10. Verbindung nach Anspruch 1, dadurch gekennzeichnet, daß R⁴ -OH bedeutet.

11. Verbindung nach Anspruch 10, dadurch gekennzeichnet, daß R¹ -C(O)CH₃ bedeutet.

12. Verbindung nach Anspruch 10, dadurch gekennzeichnet, daß R¹ H bedeutet.

13. Verbindung nach Anspruch 11 oder 12, dadurch gekennzeichnet, daß R³ H bedeutet.

14. Verbindung nach Anspruch 11 oder 12, dadurch gekennzeichnet, daß R³ -OH bedeutet.

15. Verbindung nach einem der Ansprüche 1 bis 14,dadurch gekennzeichnet, daß n eine ganze Zahl von 6 bis 14 bedeutet.

16. Verbindung nach Anspruch 15, dadurch gekennzeichnet, daß n 8 bedeutet.

17. Verbindung nach Anspruch 15, dadurch gekennzeichnet, daß n 10 bedeutet.

18. Verfahren zur Herstellung einer Verbindung gemäß einem der Ansprüche 1 bis 17, dadurch gekennzeichnet, daß eine Hefe mit der Fähigkeit, Sophoroselipide in Form eines Lactons in den Kulturüberstand zu sezernieren, in einem Kulturmedium fermentiert wird, welches neben einer weiteren Kohlenstoffquelle, wie Glycerin, Succinat oder Mono-, Di- oder Trisaccharide, ein 2-Alkanol mit einer Kettenlänge von 6 bis 30 Kohlenstoffatomen enthält, wonach die Verbindung aus der Kulturlösung isoliert und gegebenfalls einer alkalischen Hydrolyse unterworfen wird.

19. Verfahren nach Anspruch 18 zur Herstellung einer Verbindung gemäß Anspruch 15, dadurch gekennzeichnet, daß die Kettenlänge des 2-Alkanols 10 bis 18 Kohlenstoffatome beträgt.

20. Verfahren nach Anspruch 19 zur Herstellung einer Verbindung gemäß Anspruch 16, dadurch gekennzeichnet, daß als 2-Alkanol 2-Dodecanol eingesetzt wird.

21. Verfahren nach Anspruch 19 zur Herstellung einer Verbindung gemäß Anspruch 17, dadurch gekennzeichnet, daß als 2-Alkanol 2-Tetradecanol eingesetzt wird.

22. Verfahren nach einem der Ansprüche 18 bis 21, dadurch gekennzeichnet, daß eine Hefe der Gattung Candida, vorzugsweise Candida bombicola, Candida bogoriensis, Candida magnoliae, Candida gropengiesseri oder Candida apicola, fermentiert wird.

23. Verfahren nach einem der Ansprüche 18 bis 22, dadurch gekennzeichnet, daß als weitere Kohlenstoffquelle Glucose oder Saccharose eingesetzt wird.

24. Verwendung der Verbindung gemäß einem der Ansprüche 1 bis 17 als Tensid.
